# EUROPEAN PATENT SPECIFICATION

(11) **EP 0 742 829 B1**
(45) Date of publication and mention of the grant of the patent: **12.11.2003**
(21) Application number: 95907080.6
(22) Date of filing: 31.01.1995
(51) Int. Cl.: C12N 15/62, C12N 15/70, C12N 15/74, C12N 1/21, A61K 39/08

(54) **EXPRESSION OF HETEROLOGOUS PROTEINS IN ATTENUATED BACTERIA USING THE HTRA-PROMOTERS**
EXPRESSION HETEROLOGER PROTEINE IN ABGESCHWÄCHTEN BAKTERIEN MITTELS DES HTRA-PROMOTORS
EXPRESSION DE PROTEINES HETEROLOGUES DANS DES BACTERIES ATTENUEES AU MOYEN DE PROMOTEURS DU GENE HTRA

(30) Priority: 31.01.1994 GB 9401795
(43) Date of publication of application: 20.11.1996
(73) Proprietor: Acambis Research Limited, Cambridge CB1 9PT (GB)
(72) Inventor: KHAN, Mohammed Anjam University of Newcastle, Newcastle upon Tyne NE2 4HH (GB); CHATFIELD, Steven Neville Imp. College of Science, Research Unit London SW7 2AY (GB); LI,Jingli Imperial College of Science & Technology, London SW7 2AY (GB)
(74) Representative: Woods, Geoffrey Corlett
(86) International application number: GB9500196
(87) International publication number: WO95020665

(56) References cited:
- EP-A- 0 432 965
- WO-A-89/06974
- WO-A-91/15572
- WO-A-92/15689
- WO-A-94/03615
- NUCLEIC ACIDS RESEARCH, vol. 16,no. 21, 11 November 1988 IRL PRESS LIMITED,OXFORD,ENGLAND, pages 10053-10067, B. LIPINSKA ET AL. 'Sequence analysis and regulation of the htrA gene of Escherichia coli: a sigma32-independent mechanism of heat-inducible transcription'
- GENES & DEVELOPMENT, vol. 3,no. 9, September 1989 CSH LABORATORY PRESS, NEW YORK,US, pages 1462-1471, J.W. ERICKSON AND C.A. GROSS 'Identification od sigmaE subunit of Escherichia coli RNA polymerase: a second alternative sigma factor involved in high-temperature gene expression'
- BIOTECHNOLOGY, vol. 10,no. 8, August 1992 NATURE PUBL. CO.,NEW YORK, US, pages 888-892, S.N. CHATFIELD ET AL. 'Use of the nirB promoter to direct the stable expression of heterologous antigens in Salmonella oral vaccine strains: Development of a single-dose oral tetanus vaccine' cited in the application
- NATURE, vol. 351,no. 6326, 6 June 1991 MACMILLAN JOURNALS LTD., LONDON,UK, pages 456-460, C.K. STOVER ET AL. 'New use of BCG for recombinant vaccines'
- BIOLOGICAL ABSTRACTS, vol. 99, no. 007, Philadelphia, PA, US; abstract no. 095219, & FEMS MICROBIOL. LETTERS, vol. 126,no. 1, 1995 pages 97-102, P. EVEREST ET AL. 'Expression of LacZ from the htrA, nirB and groE promoters in Salmonella vaccine strain: influence of growth in mammalian'
- JOHNSON K.: 'The role of a stress-response protein in Salmonella typhimurium virulence' MOLECULAR MICROBIOLOGY vol. 5, no. 2, 1991, pages 401 - 407

## Description

This invention relates to vaccine compositions comprising attenuated bacteria containing DNA constructs comprising an inducible promoter operably linked to a DNA sequence encoding one or more heterologous proteins.

In recent years, there has emerged a new generation of live oral salmonella vaccines based upon strains of Salmonella which have been attenuated by the introduction of a non-reverting mutation in a gene in the aromatic biosynthetic pathway of the bacterium. Such strains are disclosed, for example, in EP-A-0322237. The aforesaid live oral salmonella vaccines are showing promise as vaccines for salmonellosis in man and animals, and they can also be used effectively as carriers for the delivery of heterologous antigens to the immune system. Combined salmonella vaccines have been used to deliver antigens from viruses, bacteria, and parasites, eliciting secretory, humoral and cell-mediated immune responses to the recombinant antigens. Combined salmonella vaccines show great potential as single dose oral multivaccine delivery systems [C. Hormaeche et al, FEMS Symposium No. 63, Plenum,New York; pp 71-83, 1992].

There are problems to be overcome in the development of combined salmonella vaccines. A major consideration is obtaining a high level of expression of the recombinant antigen in the salmonella vaccine so that it will be sufficient to trigger an immune response. However, unregulated high level expression of foreign antigens can be toxic and affect cell viability [I. Charles and G. Dougan, TIBTECH 8, pp 117-21, 1990], rendering the vaccine ineffective or causing loss of the recombinant DNA. Several possible solutions to this problem have been described, such as expression from plasmids carrying essential genes, "on-off" promoters or incorporation of the foreign genes into the salmonella chromosome.

An alternative approach to overcoming the aforesaid problem would be to use a promoter which is inducible in vivo, and one such promoter is the E.coli nitrite reductase promoter nirB which is induced under anaerobiosis. Vaccine compositions containing bacteria transformed with constructs comprising the nirB promoter are described in our earlier International Patent Application PCT/GB93/01617. The use of the nirB promoter in vaccine strains of bacteria is also disclosed in the article by Chatfield et al Bio/Technology, Vol. 10, 888-892 (1992).

The present invention relates to the preparation of DNA constructs containing a different inducible promoter, namely the promoter for the htrA gene which encodes a stress induced protein.

The htrA gene is described and referred to in the articles by K. Johnson et al in Mol. Microbiol 1991; 5:401-7 and Res. Microbiol. 1990, 141, 823-825, and references cited therein and is an example of a gene encoding a heatshock protein which is produced in response to a temperature increase above 42°C. Another heat shock protein is discussed in Stover et al Nature, Volume 351, 456-460 (1991) where the use of the regulatory sequences of BCG hsp60 and hsp70 genes to drive the expression of foreign antigen genes in BCG is described. However, the said regulatory sequences have only a relatively low level of homology with the htrA promoter.

The present invention provides a vaccine composition comprising an attenuated bacterium containing a DNA construct comprising the htrA promoter sequence operably linked to a DNA sequence encoding one or more heterologous proteins; and a pharmaceutically acceptable carrier.

In one embodiment, the invention provides a vaccine composition as hereinbefore defined wherein the htrA promoter sequence is operably linked to a DNA sequence encoding a fusion protein of two or more heterologous proteins.

The proteins making up the fusion may be linked by means of a flexible hinge region.

In one particular embodiment, the DNA construct comprising the htrA promoter sequence can be operably linked to a DNA sequence encoding first and second heterologous proteins wherein the first heterologous protein is an antigenic sequence comprising tetanus toxin fragment C or one or more epitopes thereof.

The first and second proteins are preferably heterologous proteins and in particular can be polypeptide immunogens; for example they may be antigenic sequences derived from a virus, bacterium, fungus, yeast or parasite. In particular, it is preferred that the first said protein is an antigenic sequence comprising tetanus toxin fragment C or epitopes thereof.

The second protein is preferably an antigenic determinant of a pathogenic organism. For example, the antigenic determinant may be an antigenic sequence derived from a virus, bacterium, fungus, yeast or parasite.

Examples of viral antigenic sequences for the first and/or second heterologous proteins are sequences derived from a type of human immunodeficiency virus (HIV) such as HIV-1 or HIV-2, the CD4 receptor binding site from HIV, for example from HIV-1 or -2, hepatitis A, B or C virus, human rhinovirus such as type 2 or type 14, Herpes simplex virus, poliovirus type 2 or 3, foot-and-mouth disease virus (FMDV), rabies virus, rotavirus, influenza virus, coxsackie virus, human papilloma virus (HPV), for example the type 16 papilloma virus, the E7 protein thereof, and fragments containing the E7 protein or its epitopes; and simian immunodeficiency virus (SIV).

Examples of antigens derived from bacteria are those derived from Bordetella pertussis (e.g. P69 protein and filamentous haemagglutinin (FHA) antigens), Vibrio cholerae, Bacillus anthracis, and E.coli antigens such as E.coli heat Labile toxin B subunit (LT-B), E.coli K88 antigens, and enterotoxigenic E.coli antigens. Other examples of antigens include the cell surface antigen CD4, Schistosoma mansoni P28 glutathione S-transferase antigens (P28 antigens) and antigens of flukes, mycoplasma, roundworms, tapeworms, Chlamydia trachomatis, and malaria parasites, eg. parasites of the genus plasmodium or babesia, for example Plasmodium falciparum, and peptides encoding immunogenic epitopes from the aforementioned antigens.

Particular antigens include the full length Schistosoma mansoni P28, and oligomers (e.g. 2, 4 and 8mers) of the immunogenic P28 aa 115-131 peptide (which contains both a B and T cell epitope), and human papilloma virus E7 protein, Herpes simplex antigens, foot and mouth disease virus antigens, simian immunodeficiency virus antigens, and the diphtheria toxin antigens, e.g. the diphtheria toxin ganglioside binding region.

As used herein, references to the htrA promoter refer to the promoter itself or a part or derivative thereof which is capable of promoting expression of a coding sequence. The preferred sequence, and which contains the htrA promoter is:

In the constructs of the present invention, the DNA sequence may encode a fusion protein of two or more proteins in which adjacent proteins are separated by a hinge region. The hinge region is a region designed to promote the independent folding of both the first and second proteins by providing both spatial and temporal separation between the domains.

The hinge region typically is a sequence encoding a high proportion of proline and/or glycine amino acids. The hinge region may be composed entirely of proline and/or glycine amino acids. The hinge region may comprise one or more glycine-proline dipeptide units.

The hinge region may, for example, contain up to about fifteen amino acids, for example at least 4 and preferably 6-14 amino acids, the number of amino acids being such as to impart flexibility between the first and second proteins.

In one embodiment, the hinge region can correspond substantially to the hinge domain of an antibody immunoglobulin. The hinge regions of IgG antibodies in particular are rich in prolines [T.E. Michaelson et al. J. Biol. Chem. 252, 883-9 1977], which are thought to provide a flexible joint between the antigen binding and tail domains.

Without wishing to be bound by any theory, the prolines are thought to form the rigid part of the hinge as the ring structure characteristic of this amino acid hinders rotation around the peptide bond that connects the proline residue with an adjacent amino acid. This property is thought to prevent proline, and adjacent residues, from adopting the ordered structure of an alpha helix or beta strand. Flexibility is thought to be imparted by glycine, the simplest amino acid, with very limited steric demands. Glycine is thought to function as a flexible elbow in the hinge. Other amino acids may be substituted for glycine, particularly those without bulky side-chains, such as alanine, serine, asparagine and threonine.

In one preferred embodiment, the hinge region is a chain of four or more amino acids defining the sequence

-[X]ₚ-Pro-[Y]_{q}-Pro-[Z]ᵣ-

wherein Pro is proline, X and Y are each glycine, or an amino acid having a non-bulky side chain; Z is any amino acid; p is a positive integer; q is a positive integer of from one to ten; and r is zero or a positive integer greater than zero.

The hinge region can be a discrete region heterologous to both the first and second proteins or can be defined by a carboxy-end portion of the first protein or an amino-end portion of the second protein.

In a most preferred aspect, the present invention provides a vaccine composition comprising a pharmaceutically acceptable carrier and an attenuated bacterium containing a DNA molecule comprising the htrA promoter operably linked to a DNA sequence encoding first and second polypeptide immunogens linked by a hinge region, wherein the first polypeptide immunogen comprises tetanus toxin fragment C or epitopes thereof.

In a further embodiment, the DNA construct comprises the htrA promoter operably linked to DNA encoding a first protein and, extending from the 3' end thereof a DNA sequence encoding the hinge region, and downstream thereof, one or more restriction endonuclease sites.

The said protein is preferably an antigenic protein as hereinbefore defined, and in particular is the TetC fragment or epitopes thereof.

Stable expression of the first and second heterologous proteins linked by the hinge region can be obtained in vivo. The heterologous proteins can be expressed in an attenuated bacterium which can thus be used as a vaccine.

The attenuated bacterium may be selected from the genera Salmonella, Bordetella, Vibrio, Haemophilus, Neisseria and Yersinia. Alternatively, the attenuated bacterium may be an attenuated strain of enterotoxigenic Escherichia coli. In particular the following species can be mentioned: S.typhi - the cause of human typhoid; S.typhimurium - the cause of salmonellosis in several animal species; S.enteritidis - a cause of food poisoning in humans; S.choleraesuis - a cause of salmonellosis in pigs; Bordetella pertussis - the cause of whooping cough; Haemophilus influenzae a cause of meningitis; Neisseria gonorrhoeae - the cause of gonorrhoea; and Yersinia - a cause of food poisoning.

Attenuation of the bacterium may be attributable to a non-reverting mutation in a gene in the aromatic amino acid biosynthetic pathway of the bacterium. There are at least ten genes involved in the synthesis of chorismate, the branch point compound in the aromatic amino acid biosynthetic pathway. Several of these map at widely differing locations on the bacterial genome, for example aroA (5-enolpyruvylshikimate-3-phosphate synthase), aroC (chorismate synthase), aroD (3-dihydroquinate dehydratase) and aroE (shikimate dehydrogenase). A mutation may therefore occur in the aroA, aroC, aroD, or aroE gene.

Preferably, however, an attenuated bacterium harbours a non-reverting mutation in each of two discrete genes in its aromatic amino acid biosynthetic pathway; or harbours a non-reverting mutation in its aromatic biosynthetic pathway and a non-reverting mutation in a regulatory gene such as htrA, OmpR or OsmC. Examples of suitable attenuated bacteria are disclosed in, for example, EP-A-0322237, and EP-A-0400958.

The vaccine compositions of the invention may comprise one or more suitable adjuvants.

The vaccine is advantageously presented in a lyophilised form, for example in a capsular form, for oral administration to a patient. Such capsules may be provided with an enteric coating comprising, for example, Eudragit "S", Eudragit "L", Cellulose acetate, Cellulose acetate phthalate or Hydroxypropylmethyl Cellulose. These capsules may be used as such; or alternatively, the lyophilised material may be reconstituted prior to administration, e.g. as a suspension. Reconstitution is advantageously effected in buffer at a suitable pH to ensure the viability of the organisms. In order to protect the attenuated bacteria and the vaccine from gastric acidity, a sodium bicarbonate preparation is advantageously administered before each administration of the vaccine. Alternatively, the vaccine may be prepared for parenteral administration, intranasal administration or intramammary administration.

Preferably, the vaccine composition is adapted for mucosal delivery, eg by oral administration; by intranasal administration or by intrabronchial administration.

The vaccine compositions of the invention may be used in prophylaxis or treatment of a host, particularly a human host but also possibly an animal host. An infection caused by a micro-organism, especially a pathogen, may therefore be prevented by administering an effective dose of a vaccine composition containing an attenuated bacterium according to the invention. The bacterium then expresses a heterologous protein or proteins capable of raising antibody to the micro-organism. The dosage employed will be dependent on various factors including the size and weight of the host, the type of vaccine formulated and the nature of the heterologous protein.

An attenuated bacterium useful in the vaccine compositions of the present invention may be prepared by transforming an attenuated bacterium with a DNA construct as hereinbefore defined. Any suitable transformation technique may be employed, such as electroporation. In this way, an attenuated bacterium capable of expressing a protein or proteins heterologous to the bacterium may be obtained. A culture of the attenuated bacterium may be grown under aerobic conditions. A sufficient amount of the bacterium is thus prepared for formulation as a vaccine, with minimal expression of the heterologous protein occurring.

The expression vector is provided with appropriate transcriptional and translational control elements including, besides the htrA promoter, a transcriptional termination site and translational start and stop codons and an appropriate ribosome binding site is provided. The vector typically comprises an origin of replication and, if desired, a selectable marker gene such as an antibiotic resistance gene. The vector may be, for example, a plasmid.

The invention will now be illustrated, but not limited, by reference to the following examples, and the accompanying drawings, in which:
Figure 1 is a schematic illustration of the construction of a plasmid pHTRA1 containing the htrA promoter in accordance with one aspect of the invention;
Figure 2 is a schematic illustration of the construction of a plasmid pHTRA2 containing the htrA promoter and DNA encoding the tetanus toxin C-fragment linked to a hinge region;

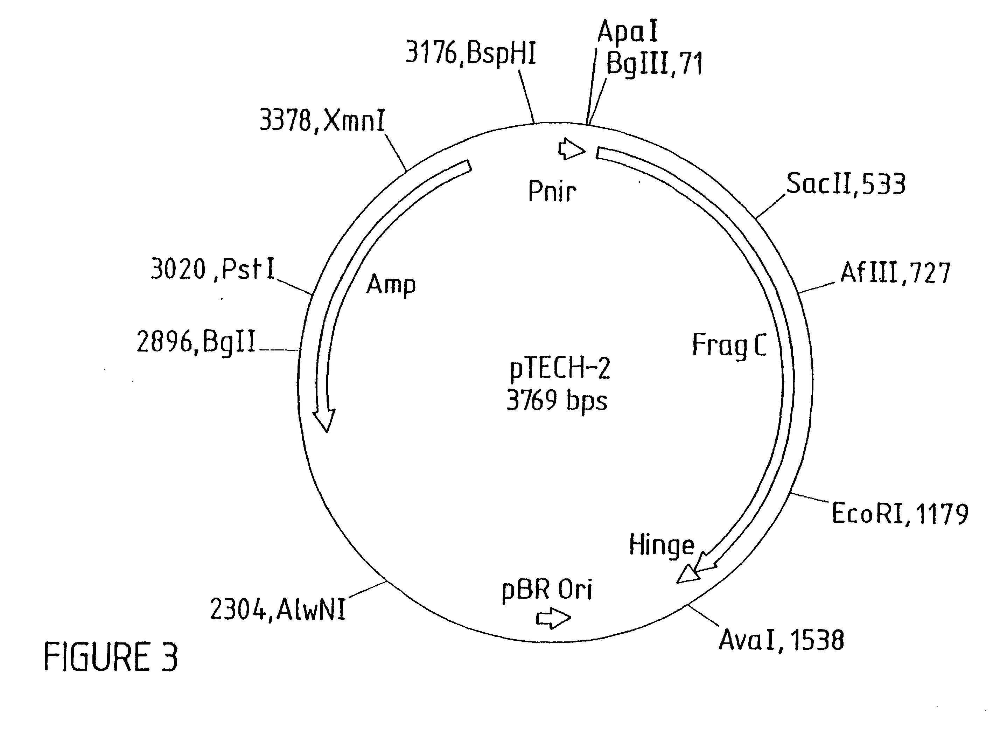

Figure 3 illustrates the structure of the plasmid pTECH2;

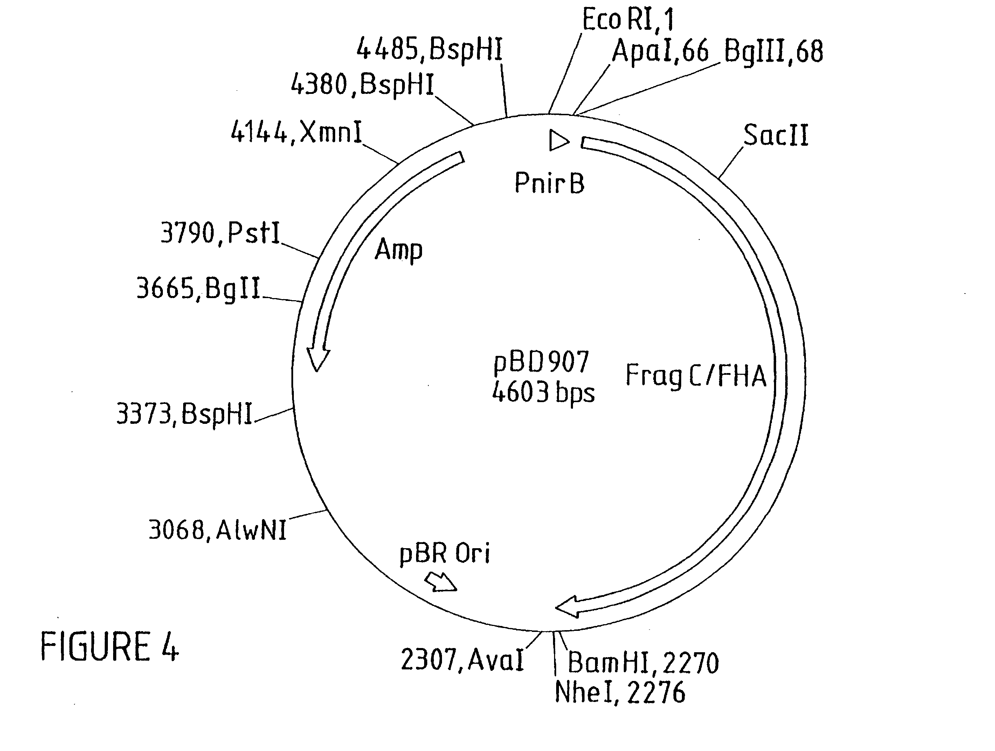

Figure 4 illustrates the structure of the intermediate plasmid pBD907;
Figure 5 shows the structure of the plasmid PHTRA1 prepared in accordance with the scheme shown in Figure 1;

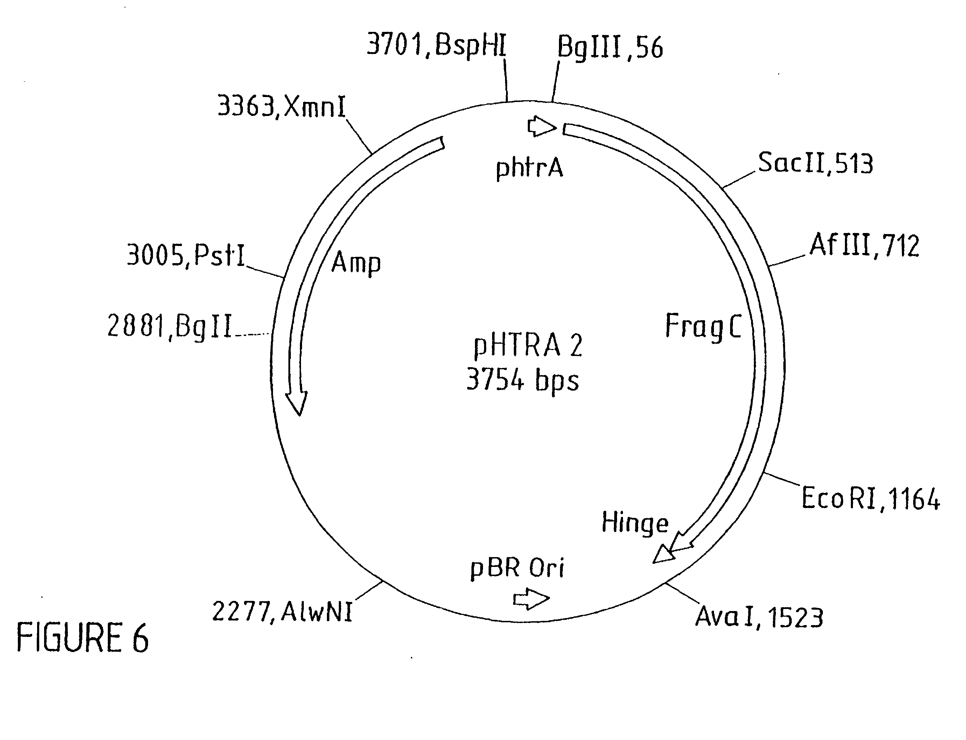

Figure 6 shows the structure of the product plasmid pHTRA2 prepared in accordance with the scheme shown in Figure 2;
Figures 7A to 7B illustrate the influence of temperature shifts on the promoters nirB, groE and hrtA; and
Figure 8 shows the expression of lacZ from htrA, nirB and groE in macrophages.

### EXAMPLE 1

### Preparation of htrA-TetC-Hinge Construct

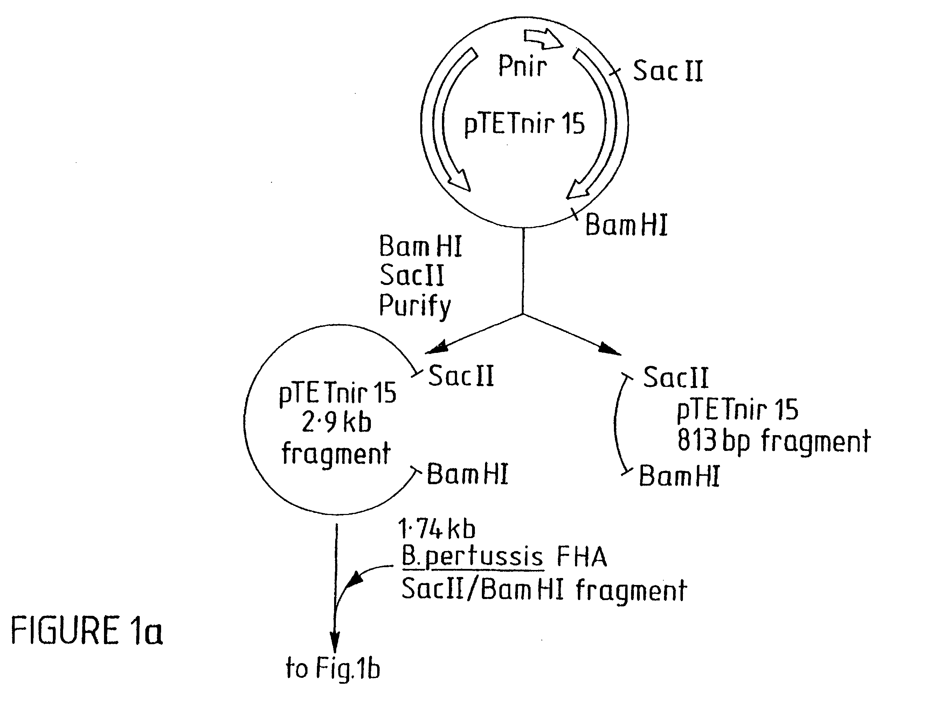

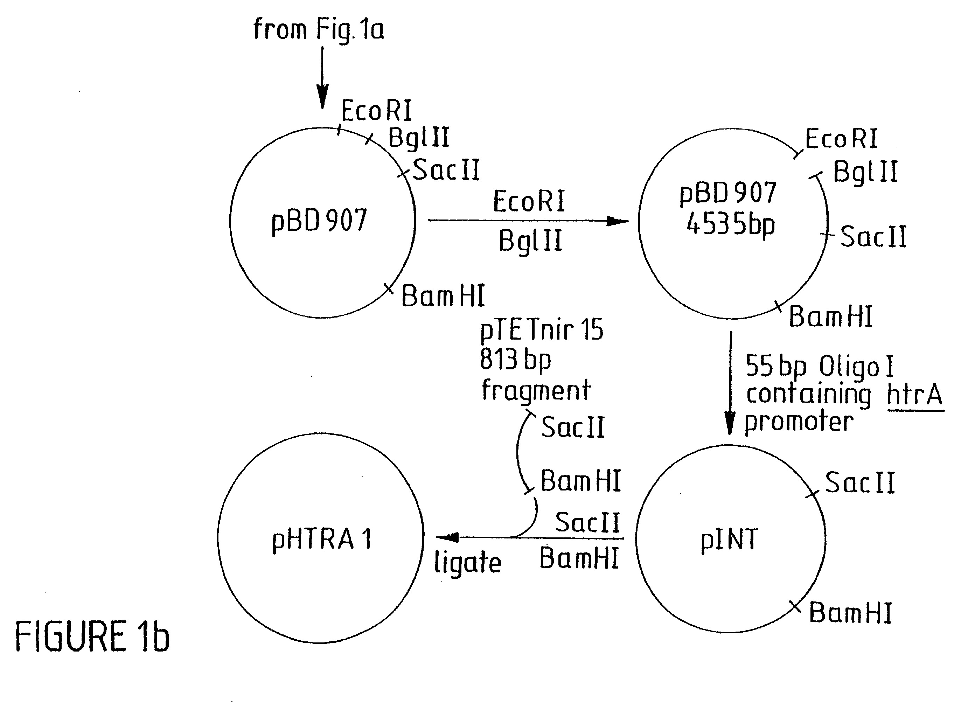

As can be seen from Figure 1, the starting material for the preparation of a vector containing the htrA promoter and genes coding for the tetanus toxin C fragment was the plasmid pTETnir15, the structure and preparation of which is disclosed in our earlier application PCT/GB93/01617 (Publication No. WO 94/03615) and references cited therein, e.g. WO-A-92159689.

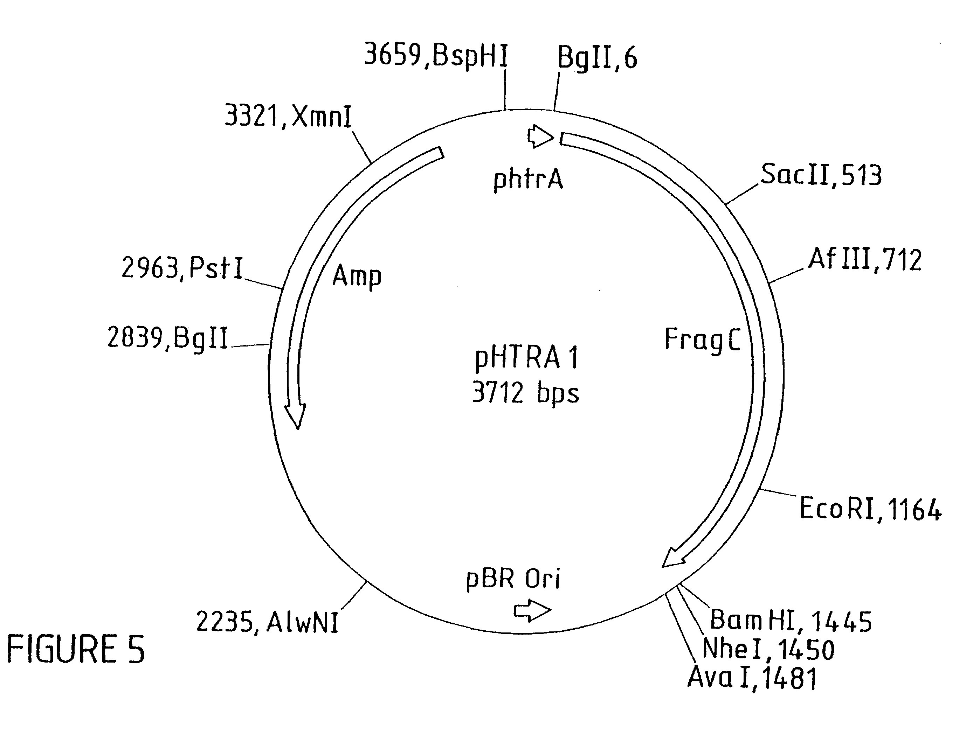

The pTETnir15 plasmid contains the nirB promoter linked to the gene encoding the C-fragment of tetanus toxin (TetC). As shown in Figure 1, pTETnir15 was digested with SacII and BamHI and the resulting 2.9kb and 813bp fragments were gel-purified. The 2.9kb fragment was ligated with a 1.74kb fragment derived from the B. pertussis filamentous haemagglutinin (FHA) gene, the fragment having the sequence shown in SEQ.ID.NO.7. The resulting plasmid was designated pBD907 and the restriction map of the plasmid is shown in Figure 5. The purpose of preparing the intermediate plasmid pBD907 was to remove the EcoRI site present in the TetC fragment in order that the nirB promoter sequence could be replaced by the htrA promoter sequence. This was achieved by digesting plasmid pBD907 with EcoRI and BglII. The resulting 4535bp fragment was gel-purified and ligated with the following 55bp oligonucleotides containing the htrA promoter:

The presence of the promoter in the resulting intermediate plasmid pINT was confirmed by DNA sequencing. The plasmid pINT was then digested with SacII and BamHII and ligated to the 813bp fragment from pTETnir15 to form plasmid pHTRA1. The DNA sequence of pHTRA1 is shown in SEQ.ID.NO.4; the htrA region which is defined by the first 55 base pairs, has the sequence

In relation to SEQ.ID.N0.4, GAACTT is -35 box, and TCTGA is -10 box. At 513 and 2235 base pairs respectively are SacII and AlwN 1 restriction sites.

Plasmid pHTRA1 was used to transform Salmonella typhimurium strain BRD509 (deposited under accession number NCTC 12716 on 12th October 1992) and the resulting strain, designated BRD935, was checked for expression of TetC fragment by standard methods. Strain BRD935 has been deposited at the National Collection of Type Cultures, Colindale, United Kingdom on 27th January 1995 under the accession number 12883).

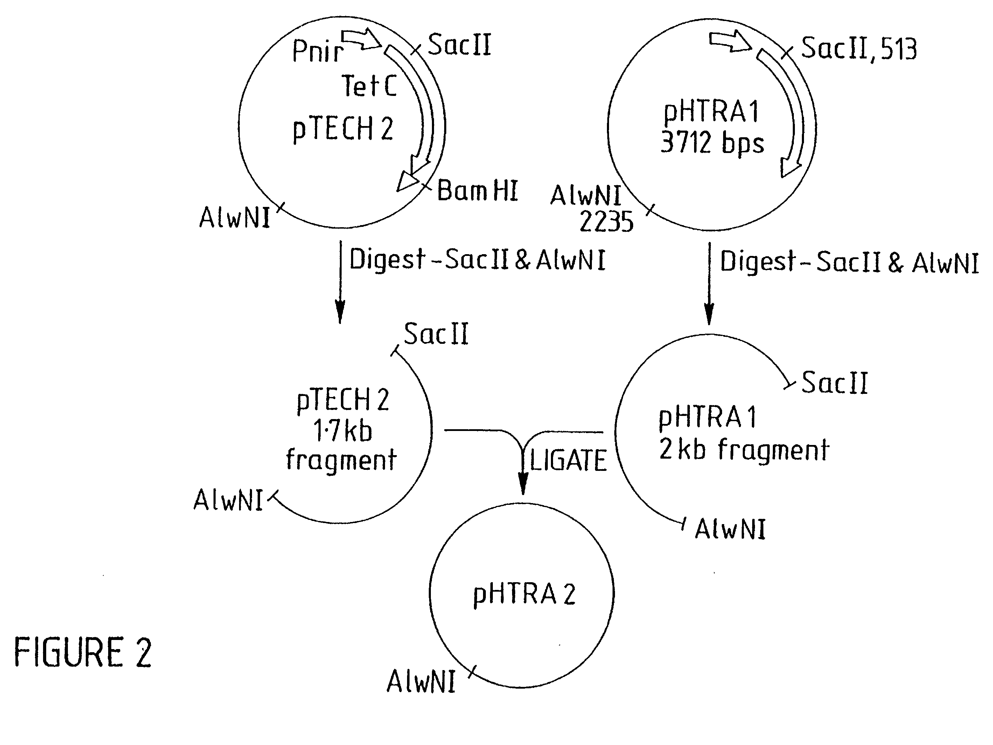

As shown in Figure 2, plasmid pHTRA1 was used to prepare a modified construct in which a "hinge" region is present at the C-terminal of the TetC fragment. The nucleotide sequence representing the "hinge" region was obtained from plasmid pTECH2 which has the DNA sequence set forth in SEQ.ID.N0.5, and possesses SacII and AlwNI restriction sites at positions 533 and 2304 respectively. The preparation of this plasmid is disclosed in our earlier Application PCT/GB93/01617.

The pTECH2 plasmid comprises the nirB promoter region linked to the tetanus toxin C fragment which, at its 3' terminal, is linked via a BamHI restriction site to a hinge region encoded a Gly-Pro-Gly-Pro repeat motif along with a number of restriction sites allowing the insertion of genes encoding further polypeptides. A 1.7kb fragment encoding the hinge region and part of the tetanus toxin C fragment region was removed from pTECH2 through digestion with SacII and AlwNI and purified. The DNA sequence of the resulting fragment is shown in SEQ.ID.NO.6.

Plasmid pHTRA1, which encodes the htrA promoter and the tetanus toxin C fragment, but includes no hinge, was digested with SacII and AlwNI and the resulting 2kb fragment was gel-purified.

The 1.7kb fragment (SEQ.ID.NO.6) from pTECH2 and the 2kb fragment from the pHTRA1 were ligated to form plasmid pHTRA2 which incorporates a htrA promoter operably linked to the gene for tetanus toxin C fragment, having at the 3' terminal thereof the hinge region.

An attenuated Salmonella typhimurium strain was transformed with vector pHTRA2 and after selection by means of standard techniques, the salmonella strain BRD1062, harbouring the plasmid pHTRA2 was isolated.

Plasmid pHTRA2 serves as an intermediate for the preparation of constructs coding for a fusion protein linked by the hinge region. Thus, in accordance with the techniques described in our earlier Application No. PCT/GB93/01617, further proteins can be cloned into the restriction endonuclease sites on the hinge region.

### MATERIALS AND METHODS

### Bacterial Strains

E.coli HB101 and BRD509 (an attenuated S. typhimurium aroA aroD strain (Deposited under accession number NCTC 12716 on 12th October 1992...) were used throughout the experiments. The bacteria were grown in Luria broth (LB) or LB solidified with 1.6% w/v agar supplemented with appropriate antibiotics.

### DNA Manipulations

Plasmid DNA was purified by the alkaline lysis method (R. Maniatis, et al., 1982 Molecular Cloning - A Laboratory Manual, Cold Spring Harbor Laboratory, New York). Restricted DNA fragments were purified from agar gels by the method of Tautz and Rentz (1983, "An optimised freeze-squeeze method for the recovery of DNA Fragments from agarose gels". Analytical Biochem., 132, 14-19). Restriction enzymes were supplied by Boehringer Mannheim, Germany and New England Biolabs, USA and were used according to the manufacturer's instructions.

### DNA Sequencing

DNA for double stranded sequencing was isolated by the method of Stephen et al. (1990, A Rapid Method for Isolating High Quality Plasmid DNA Suitable for DNA Sequencing, Nucleic Acid Research, 18, No. 24, p 7463). Sequencing was carried out using a Sequenase Version 2 kit (USB) and was used according to the manufacturer's instructions.

### Oligonucleotides

These were synthesised on a SAM1 oligonucleotide synthesiser (Biolabs, UK).

### EXAMPLE 2

### Preparation of htrA-lacZ Construct

The properties of the htrA promoter were composed with two other inducible promotors, namely the nirB and groE promoters.

### Sub-cloning of lacZ downstream to nirB, htrA and groE promoters

A DNA fragment encoding a promoterless lacZ gene was purified from plasmid pMAC1871 (Pharmacia) by the low melting point agarose technique, following cleavage of the plasmid with restriction enzymes SalI and BamHI [14]. Plasmids pTETnir-15 [S.N. Chatfield *et al*, Bio/Technology 10, 888-892] and pTEThtrA-1 harbouring the nirB and htrA promoters respectively, were digested with SalI and BamHI endonucleases and the purified lacZ encoding fragment was cloned, in-frame, downstream of the promoters. Plasmid pRZ-PES was used to measure expression of β-galactosidase (β-gal) from the groE promoter. pRZ-PES contains the E. coli groE-operon promoter upstream of groES and lacZ genes. It was constructed by subcloning a 2.1 Kb EcoRI-HindIII fragment carrying the operon from plasmid pOF39 [O. Fayet *et al* J. Bacteriol. 171, 1379-1385 (1989)] into pUC19. A novel BglII site was then introduced between the groES and groEL genes using site directed mutagenesis. The EcoRI-BglII fragment carrying the groE promoter and groES gene was cloned into EcoRI-BamHI cut promoter-probe plasmid pRF5255 [P.F. Lambert *et al* J. Bacteriol. 162, 441-444 (1985)] to give plasmid pRZ-PES. Plasmids, prepared in S. typhimurium LB5010 (r⁻m⁺) [L.R. Bullas *et al J.* Bacteriol. 256, 471-474 (1983)], were introduced into S. typhimurium strain BRD915 (S. typhimurium SL1344 htrA) [S.N. Chatfield *et al* Microbial Pathog. 12, 145-151 (1992)] using electroporation. Lac positive recombinants were screened on L agar plates containing ampicillin and X-gal.

### Effect of changes in environmental conditions on lacZ expression

Bacterial strains harbouring the recombinant plasmids were grown overnight in L-broth, with skaing at 30°C. The cultures were diluted 1:50 and growth was allowed to continue for an additional 3 hours at 30°C until an OD₆₀₀ of 2.8-3.4 was reached. 0.2 ml of each culture was stored at 4°C and used to determine the base-line of β-gal activity. The remaining portions of the cultures were then shifted to different growth conditions as described below and samples were taken at 0, 2, 4, 6 and 24 hours, unless otherwise specified. At each time point the OD₆₀₀ was determined and the bacteria were stored at 4°C prior to performing a β-gal assay.

### Measuring expression in infected HEp-2, Caco-2 and THP 1-macrophage cell lines

Cells were seeded at approximately 10⁵ cell per well in twenty four well plates and grown overnight in Dolbecco's modified Eagles medium, without phenol red (ICN Flow), supplemented with 10% (vol/vol) fetal calf serum and 2 mM glutamine at 37°C, in an atmosphere of 5% CO₂. 10⁸ CFU bacteria of the diluted overnight culture were added to the tissue culture cells and incubated at 30°C. At various time points samples of the tissue culture medium were taken to measure β-gal activity in the extra cellular bacteria. The numbers of bacteria in each sample were determined by viable count and the corresponding OD₆₀₀ was determined using a standard curve. Infected cells were washed with phosphate buffer saline (PBS) and incubated for an additional hour in the presence of 200 mg/ml of gentamicin to kill extra cellular bacteria. Thereafter, the cells were lysed using sterile distilled water and vigorous pipetting. β-gal activity was determined for each cell lysate. The numbers of bacteria in each lysate were determined by viable count and the corresponding OD₅₀₀ values were determined using a standard curve.

### RESULTS

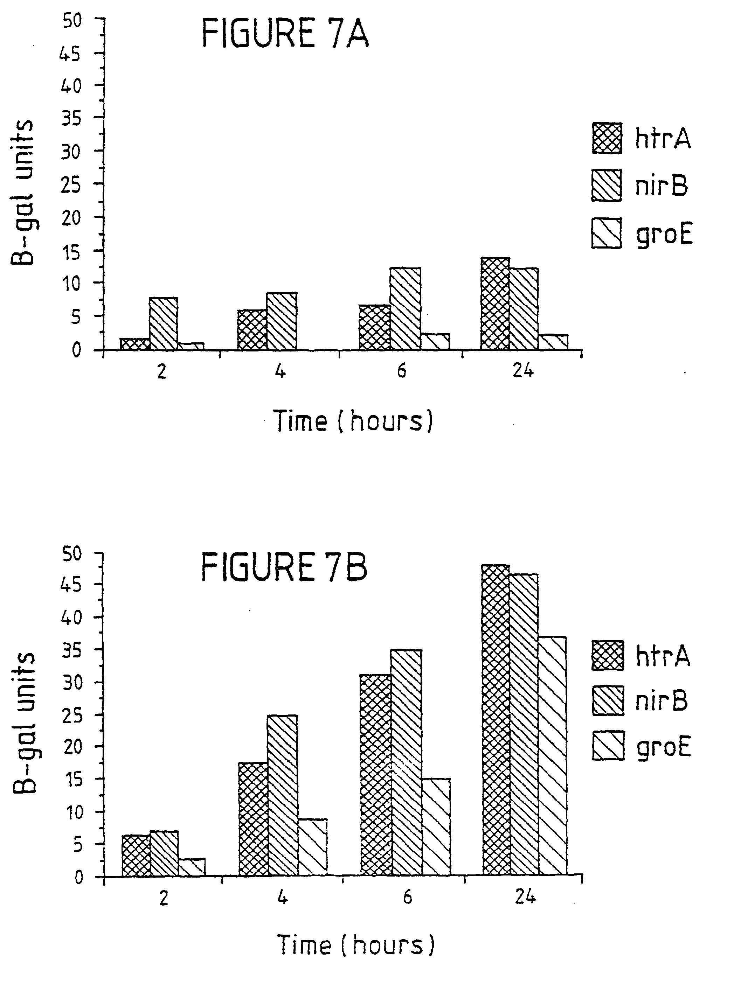

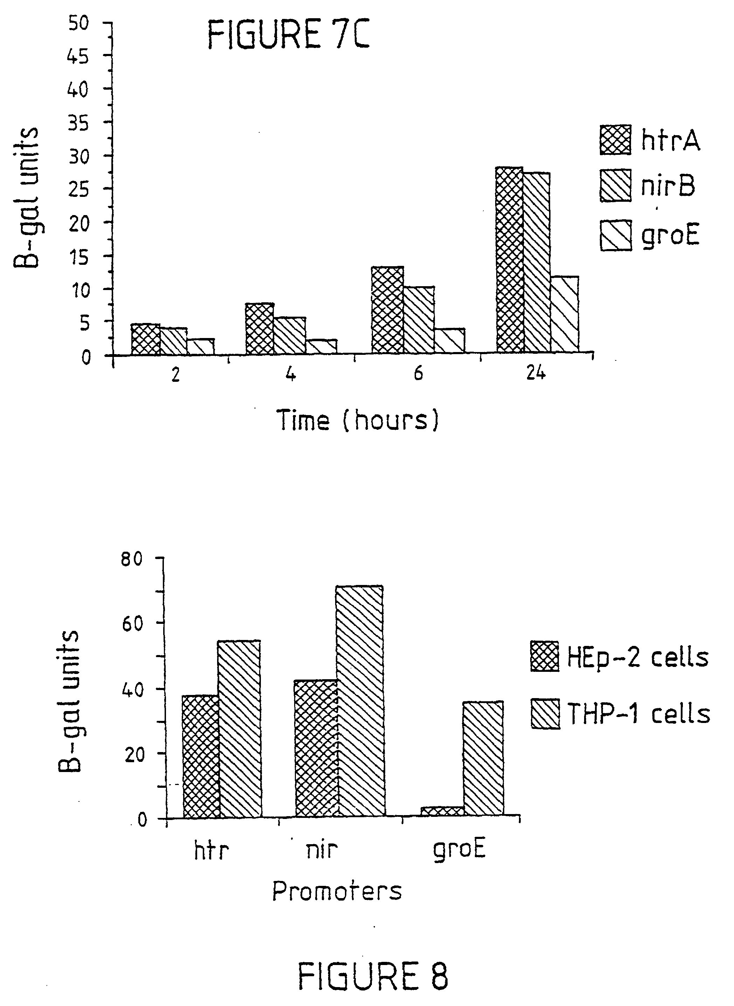

Expression from each of the promoters selected for this study is sensitive to changes in environmental conditions. nirB has been shown previously to respond to changes in anaerobicity. Initial experiments were performed to assess the levels of lacZ expression from each of the promoters, resident on similar multicopy plasmids, harboured within Salmonella vaccine strain BRD915. The influence of temperature shifts on the different promoters is shown in Figure 7. Temperature shifts from 30°C to 37°C (Figure 7A) resulted in an increase in β-gal enzyme units when lacZ was expressed from the nirB and htrA promoters. No significant increase in β-gal units was detected from the groE promoter. A temperature shift from 30°C to 42° resulted in an increase in the number of β-gal units from all three promoters. The rate of the increase in the level of β-gal was faster from htrA and nirB compared with groE (Figure 7B). Temperature shifts from 37°C to 42°C resulted in the induction of both nirB and htrA promoters, with more moderate increase in β-gal units from groE promoter (Figure 7C).

Expression of β-gal from the different promoters was also tested by selecting for bacteria that had entered eukaryotic cells. HEp-2, Caco-2 and THP-1 macrophage cell lines were infected with 10⁸ bacteria and incubated at 30°C. The number of β-gal units, determined three hours after infection of HEp-2, showed that expression of lacZ from both htrA and nirB promoters was significantly enhanced (Figure 2). However there was no detectable increase in lacZ expression from groE promoter. Similar results were obtained in infected Caco-2 cells (not shown). In contrast, in the macrophage's intracellular environment, all three promoters were induced (Figure 8). nirB promoter was most affected and groE promoter was least affected (Figure 8). When the number of β-gal units in the extra-cellular medium of either cell line was determined, no increase in the enzyme activity was seen (not shown).

Since growth within macrophages was found to influence expression from all three promoters, their sensitivity to hydrogen peroxide, commonly found within the phagosome of macrophages, was monitored. Incubating the bacteria at 30°C in 100 µM hydrogen peroxide resulted in no significant effect on the groE and nirB promoters. In contrast, the level of β-gal was increased from the htrA promoter reaching 10 U above base-line level by 4 hours. This was followed by a rapid decrease to base-line levels by 6 hours (not shown). Constitutive expression of lacZ from plasmid pLK [M. Szabo *et al* J. Bacteriol. 174, 7245-7252] was not significantly affected by any of the environmental conditions (not shown).

In this study three environmentally regulated promoters were used to express lacZ gene under different growth conditions. The promoters are representatives of three classes of inducible bacterial promoters: the anaerobically inducible E. coli nirB, the σ^{E} dependent htrA and σ³²-dependent groE. Expression from the nirB promoter is dependent on the transcription factor FNR which binds between positions -52 and -30 upstream from transcription start. In some cases FNR dependent transcription is modulated together with a second transcription factor NarL. However, plasmid pTETnir-15 used here contains only the FNR dependent bind site.

Bacterial respond to environmental stress conditions by rapid change in the rate of synthesis of many proteins. In many cases the transit induction rapidly adjusts the protein levels to a new steady state. In this study we tested the influence of environmental conditions on the level of β-gal. We found that temperature shift had a greater effect on htrA promoters compared with groE. This result is in line with the fact that in vivo the htrA promoter is induced before groE (which is σ³²-dependent) and together with σ³², by σ^{E} containing RNA polymerase [11, 12]. Surprisingly, the nirB promoter was also enhanced by elevated temperature. Although it is possible that at high temperature the concentration of oxygen in the growing media is reduced the fact that the temperature shift from 30°C to 37°C brings rapid increase in the β-gal units expressed from the nirB promoter may suggest that FNR, like other stress protein modulators, responds to a number of environmental stimuli. Similarly, htrA was also induced under anaerobic growth conditions, and therefore it seems that this promoter is either being regulated by factors other than σ^{E}, or that σ^{E} is being activated also at low oxygen tension.

For S. typhimurium to retain virulence the bacteria has to be able to survive in macrophages. This survival is dependent on the ability of the bacteria to tolerate a range of toxic killing machanisms including the production of hydrogen peroxide. Unlike E. coli htrA mutants, S. typhimurium htrA mutants have been found previously not to be killed by elevated temperature, but rather to have impaired ability to survive significant levels of hydrogen peroxide. Interestingly, the htraA promoter was the only one of the test promoters whose expression was increased in the presence of hydrogen peroxide.

In order to determine the influence of the intracellular environment, the level of expression from the three different promoters was monitored after Salmonella harbouring the test plasmids had entered a number of different cultured eukaryotic cell lines. Bacteria were grown in vitro and used to infect eukaryotic cells at 30°C since a temperature shift from 30°C to 37°C dramatically induced both the htrA and the nirB promoters. We found that while the level of β-gal expression from both the nirB and htrA promoters increased in all the cell lines tested, groE promoter was induced only in infected macrophages.

## Claims

1. A vaccine composition comprising an attenuated bacterium containing a DNA construct comprising the htrA promoter sequence operably linked to a DNA sequence encoding one or more heterologous proteins; and a pharmaceutically acceptable carrier.

2. A vaccine composition according to Claim 1 wherein the htrA promoter sequence is operably linked to a DNA sequence encoding a fusion protein of two or more proteins.

3. A vaccine composition according to claim 2 wherein the proteins making up the fusion are linked by means of a flexible hinge region.

4. A vaccine composition according to claim 3 wherein the hinge region is a chain of four or more amino acids defining the sequence -[X]ₚ-Pro-[Y]_{q}-Pro-[Z]ᵣ-wherein Pro is proline, X and Y are each glycine, or an amino acid having a non-bulky side chain; Z is any amino acid; p is apositive integer; q is a positive integer from one to ten; and r is zero or a positive integer greater than zero.

5. A vaccine composition according to any one of claims 2, 3 and 4 wherein the htrA promoter is operably linked to a DNA sequence encoding first and second heterologous proteins wherein the first heterologous protein is an antigenic sequence comprising the tetanus toxin fragment C or one or more epitopes thereof.

6. A vaccine composition according to any one of the preceding claims which is adapted for oral administration or intranasal administration.

7. A vaccine composition according to any one of the preceding claims in which the attenuated bacterium is attenuated by virtue of having a non-reverting mutation in a gene in its aromatic amino acid biosynthetic pathway of the bacterium.

8. A vaccine composition according to claim 7 wherein said attenuated bacterium has additionally, a non-reverting mutation in the htrA gene.

9. A vaccine composition according to claim 7 or 8 wherein the attenuated bacterium harbours a non-reverting mutation in each of two discrete genes in its aromatic amino acid biosynthetic pathway.

10. A vaccine composition according to claim 7, 8 or 9, wherein said gene or each of said genes in the aromatic amino acid biosynthetic pathway is selected from aroA, aroC, aroD and aroE.

11. A vaccine composition according to claim 9 wherein said two discrete genes are aroA and aroD.

## Patentansprüche

1. Impfstoffzusammensetzung, die ein attenuiertes Bakterium umfasst, welches ein DNA Konstrukt enthält, das die htrA Promotorsequenz in funktioneller Verknüpfung zu einer DNA Sequenz, die für ein oder mehrere heterologe Proteine kodiert umfasst, sowie einen pharmazeutisch verträglichen Trägerstoff.

2. Impfstoffzusammensetzung nach Anspruch 1, wobei die htrA Promotorsequenz funktionell mit einer DNA Sequenz verknüpft ist, die für ein Fusionsprotein zweier oder mehrerer Proteine kodiert.

3. Impfstoffzusammensetzung nach Anspruch 2, wobei die die Fusion bildenden Proteine über eine flexible Scharnierregion verbunden sind.

4. Impfstoffzusammensetzung nach Anspruch 3, wobei die Scharnierregion eine Kette von vier oder mehr Aminosäuren darstellt, welche die Sequenz -[X]ₚ-Pro-[Y]_{q}-Pro-[Z]ᵣ- definiert, wobei Pro Prolin ist, X und Y jeweils Glycin oder eine Aminosäure darstellen, die eine nicht-sperrige Seitenkette aufweist, Z eine beliebige Aminosäure ist, p eine positive ganze Zahl ist, q eine positive ganze Zahl von eins bis zehn und r null oder eine positive ganze Zahl größer als null darstellen.

5. Impfstoffzusammensetzung nach einem der Ansprüche 2, 3 und 4, wobei der htrA Promotor funktionell mit einer DNA Sequenz verknüpft ist, die für ein erstes und zweites heterologes Protein kodiert, wobei das erste heterologe Protein eine antigene Sequenz darstellt, die das Tetanustoxinfragment C oder eines oder mehrerer Epitope davon umfasst.

6. Impfstoffzusammensetzung nach einem der vorhergehenden Ansprüche, die zur oralen oder intranasalen Verabreichung angepasst ist.

7. Impfstoffzusammensetzung nach einem der vorhergehenden Ansprüche, wobei das attenuierte Bakterium aufgrund einer nicht revertierenden Mutation in einem Gen des Biosynthesewegs aromatischer Aminosäuren des Bakteriums attenuiert ist.

8. Impfstoffzusammensetzung nach Anspruch 7, wobei das attenuierte Bakterium zusätzlich eine nicht revertierende Mutation des htrA Gens aufweist.

9. Impfstoffzusammensetzung nach Anspruch 7 oder 8, wobei das attenuierte Bakterium eine nicht revertierende Mutation in jedem zweier eigenständiger Gene des Biosynthesewegs aromatischer Aminosäuren beherbergt.

10. Impfstoffzusammensetzung nach Anspruch 7, 8 oder 9, wobei das Gen oder jedes der Gene des Biosynthesewegs aromatischer Aminosäuren ausgewählt ist aus aroA, aroC, aroD und aroE.

11. Impfstoffzusammensetzung nach Anspruch 9, wobei die beiden eigenständigen Gene aroA und aroD sind.

## Revendications

1. Composition de vaccin comprenant une bactérie atténuée contenant une construction d'ADN comprenant la séquence de promoteur htrA liée de manière fonctionnelle à une séquence d'ADN codant pour une ou plusieurs protéines hétérologues ; et un support pharmaceutiquement acceptable.

2. Composition de vaccin selon la revendication 1, dans laquelle la séquence de promoteur htrA est liée de manière fonctionnelle à une séquence d'ADN codant pour une protéine de fusion de deux protéines ou plus.

3. Composition de vaccin selon la revendication 2, dans laquelle les protéines constituant la fusion sont liées au moyen d'une région charnière flexible.

4. Composition de vaccin selon la revendication 3, dans laquelle la région charnière est une chaîne de quatre acides aminés ou plus définissant la séquence -[X]ₚ-Pro-[Y]_{q}-Pro-[Z]ᵣ- dans laquelle Pro est une proline, X et Y sont chacun une glycine, ou un acide aminé ayant une chaîne latérale non volumineuse ; z est un acide aminé quelconque ; p est un nombre entier positif ; q est un nombre entier positif de un à dix ; et r est zéro ou un nombre entier positif plus grand que zéro.

5. Composition de vaccin selon l'une quelconque des revendications 2, 3 et 4, dans laquelle le promoteur htrA est lié de manière fonctionnelle à une séquence d'ADN codant pour une première et une seconde protéines hétérologues dans lesquelles la première protéine hétérologue est une séquence antigénique comprenant le fragment C de la toxine du tétanos ou un épitope ou plus de celui-ci.

6. Composition de vaccin selon l'une quelconque des revendications précédentes qui est adaptée à une administration orale ou à une administration intranasale.

7. Composition de vaccin selon l'une quelconque des revendications précédentes, dans laquelle la bactérie atténuée est atténuée du fait qu'elle a une mutation non réversible dans un gène de la voie de biosynthèse d'acides aminés aromatiques de la bactérie.

8. Composition de vaccin selon la revendication 7, dans laquelle ladite bactérie atténuée a en outre une mutation non réversible dans le gène htrA.

9. Composition de vaccin selon la revendication 7 ou 8, dans laquelle la bactérie atténuée contient une mutation non réversible dans chacun de deux gènes distincts dans sa voie de biosynthèse d'acides aminés aromatiques.

10. Composition de vaccin selon la revendication 7, 8 ou 9, dans laquelle ledit gène ou chacun desdits gènes dans la voie de biosynthèse d'acides aminés aromatiques est choisi parmi aroA, aroC, aroD et aroE.

11. Composition de vaccin selon la revendication 9, dans laquelle lesdits deux gènes distincts sont aroA et aroD.


### SEQUENCE LISTING

(1) GENERAL INFORMATION:
   (i) APPLICANT:
      (A) NAME: MEDEVA HOLDINGS BV
      (B) STREET: CHURCHILL-LAAN 223
      (C) CITY: AMSTERDAM
      (E) COUNTRY: THE NETHERLANDS
      (F) POSTAL CODE (ZIP): 1078 ED
   (ii) TITLE OF INVENTION: VACCINES
   (iii) NUMBER OF SEQUENCES: 7
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (vi) PRIOR APPLICATION DATA:
      (A) APPLICATION NUMBER: GB 9401795.1
      (B) FILING DATE: 31-JAN-1994
(2) INFORMATION FOR SEQ ID NO: 1:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: Salmonella typhimurium
   (ix) FEATURE:
      (A) NAME/KEY: promoter
      (B) LOCATION: 1..55
   (xi) SEQUENCE DESCRIPTION: SEQ ID NO: 1:
   AATTCTATTC CGGAACTTCG CGTTATAAAA TGAATCTGAC GTACACAGCA ATTTA 55
(2) INFORMATION FOR SEQ ID NO: 2:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 2:
   AATTCTATTC CGGAACTTCG CGTTATAAAA TGAATGTGAC GTACACAGCA ATTTA 55
(2) INFORMATION FOR SEQ ID NO: 3:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: YES
   (xi) SEQUENCE DESCRIPTION: SEQ ID NO: 3:
   GATAAGGCCT TGAAGCGCAA TATTTTACTT ACACTGCATG TGTCGTTAAA TCTAG 55
(2) INFORMATION FOR SEQ ID NO: 4:
   (i) SEQUENCE CHARACTERISTICS:
      (A) LENGTH: 3712 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iii) ANTI-SENSE: NO
   (ix) FEATURE:
      (A) NAME/KEY: htrA promoter
      (B) LOCATION: 1..55
   (ix) FEATURE:
      (A) NAME/KEY: SacII restriction site
      (B) LOCATION: 513
   (ix) FEATURE:
      (A) NAME/KEY: AlwN 1 restriction site
      (B) LOCATION: 2235
   (xi) SEQUENCE DESCRIPTION: SEQ ID NO: 4:
   AATTCTATTC CGGAACTTCG CGTTATAAAA TGAATCTGAC GTACACAGCA ATTTAGATCT 60
   TAATCATCCA CAGGAGACTT TCTGATGAAA AACCTTGATT GTTGGGTCGA CAACGAAGAA 120
   GACATCGATG TTATCCTGAA AAAGTCTACC ATTCTGAACT TGGACATCAA CAACGATATT 180
   ATCTCCGACA TCTCTGGTTT CAACTCCTCT GTTATCACAT ATCCAGATGC TCAATTGGTG 240
   CCGGGCATCA ACGGCAAAGC TATCCACCTG GTTAACAACG AATCTTCTGA AGTTATCGTG 300
   CACAAGGCCA TGGACATCGA ATACAACGAC ATGTTCAACA ACTTCACCGT TAGCTTCTGG 360
   CTGCGCGTTC CGAAAGTTTC TGCTTCCCAC CTGGAACAGT ACGGCACTAA CGAGTACTCC 420
   ATCATCAGCT CTATGAAGAA ACACTCCCTG TCCATCGGCT CTGGTTGGTC TGTTTCCCTG 480
   AAGGGTAACA ACCTGATCTG GACTCTGAAA GACTCCGCGG GCGAAGTTCG TCAGATCACT 540
   TTCCGCGACC TGCCGGACAA GTTCAACGCG TACCTGGCTA ACAAATGGGT TTTCATCACT 600
   ATCACTAACG ATCGTCTGTC TTCTGCTAAC CTGTACATCA ACGGCGTTCT GATGGGCTCC 660
   GCTGAAATCA CTGGTCTGGG CGCTATCCGT GAGGACAACA ACATCACTCT TAAGCTGGAC 720
   CGTTGCAACA ACAACAACCA GTACGTATCC ATCGACAAGT TCCGTATCTT CTGCAAAGCA 780
   CTGAACCCGA AAGAGATCGA AAAACTGTAT ACCAGCTACC TGTCTATCAC CTTCCTGCGT 840
   GACTTCTGGG GTAACCCGCT GCGTTACGAC ACCGAATATT ACCTGATCCC GGTAGCTTCT 900
   AGCTCTAAAG ACGTTCAGCT GAAAAACATC ACTGACTACA TGTACCTGAC CAACGCGCCG 960
   TCCTACACTA ACGGTAAACT GAACATCTAC TACCGACGTC TGTACAACGG CCTGAAATTC 1020
   ATCATCAAAC GCTACACTCC GAACAACGAA ATCGATTCTT TCGTTAAATC TGGTGACTTC 1080
   ATCAAACTGT ACGTTTCTTA CAACAACAAC GAACACATCG TTGGTTACCC GAAAGACGGT 1140
   AACGCTTTCA ACAACCTGGA CAGAATTCTG CGTGTTGGTT ACAACGCTCC GGGTATCCCG 1200
   CTGTACAAAA AAATGGAAGC TGTTAAACTG CGTGACCTGA AAACCTACTC TGTTCAGCTG 1260
   AAACTGTACG ACGACAAAAA CGCTTCTCTG GGTCTGGTTG GTACCCACAA CGGTCAGATC 1320
   GGTAACGACC CGAACCGTGA CATCCTGATC GCTTCTAACT GGTACTTCAA CCACCTGAAA 1380
   GACAAAATCC TGGGTTGCGA CTGGTACTTC GTTCCGACCG ATGAAGGTTG GACCAACGAC 1440
   TAAGGATCCG CTAGCCCGCC TAATGAGCGG GCTTTTTTTT CTCGGGCAGC GTTGGGTCCT 1500
   GGCCACGGGT GCGCATGATC GTGCTCCTGT CGTTGAGGAC CCGGCTAGGC TGGCGGGGTT 1560
   GCCTTACTGG TTAGCAGAAT GAATCACCGA TACGCGAGCG AACGTGAAGC GACTGCTGCT 1620
   GCAAAACGTC TGCGACCTGA GCAACAACAT GAATGGTCTT CGGTTTCCGT GTTTCGTAAA 1680
   GTCTGGAAAC GCGGAAGTCA GCGCTCTTCC GCTTCCTCGC TCACTGACTC GCTGCGCTCG 1740
   GTCGTTCGGC TGCGGCGAGC GGTATCAGCT CACTCAAAGG CGGTAATACG GTTATCCACA 1800
   GAATCAGGGG ATAACGCAGG AAAGAACATG TGAGCAAAAG GCCAGCAAAA GGCCAGGAAC 1860
   CGTAAAAAGG CCGCGTTGCT GGCGTTTTTC CATAGGCTCC GCCCCCCTGA CGAGCATCAC 1920
   AAAAATCGAC GCTCAAGTCA GAGGTGGCGA AACCCGACAG GACTATAAAG ATACCAGGCG 1980
   TTTCCCCCTG GAAGCTCCCT CGTGCGCTCT CCTGTTCCGA CCCTGCCGCT TACCGGATAC 2040
   CTGTCCGCCT TTCTCCCTTC GGGAAGCGTG GCGCTTTCTC AATGCTCACG CTGTAGGTAT 2100
   CTCAGTTCGG TGTAGGTCGT TCGCTCCAAG CTGGGCTGTG TGCACGAACC CCCCGTTCAG 2160
   CCCGACCGCT GCGCCTTATC CGGTAACTAT CGTCTTGAGT CCAACCCGGT AAGACACGAC 2220
   TTATCGCCAC TGGCAGCAGC CACTGGTAAC AGGATTAGCA GAGCGAGGTA TGTAGGCGGT 2280
   GCTACAGAGT TCTTGAAGTG GTGGCCTAAC TACGGCTACA CTAGAAGGAC AGTATTTGGT 2340
   ATCTGCGCTC TGCTGAAGCC AGTTACCTTC GGAAAAAGAG TTGGTAGCTC TTGATCCGGC 2400
   AAACAAACCA CCGCTGGTAG CGGTGGTTTT TTTGTTTGCA AGCAGCAGAT TACGCGCAGA 2460
   AAAAAAGGAT CTCAAGAAGA TCCTTTGATC TTTTCTACGG GGTCTGACGC TCAGTGGAAC 2520
   GAAAACTCAC GTTAAGGGAT TTTGGTCATG AGATTATCAA AAAGGATCTT CACCTAGATC 2580
   CTTTTAAATT AAAAATGAAG TTTTAAATCA ATCTAAAGTA TATATGAGTA AACTTGGTCT 2640
   GACAGTTACC AATGCTTAAT CAGTGAGGCA CCTATCTCAG CGATCTGTCT ATTTCGTTCA 2700
   TCCATAGTTG CCTGACTCCC CGTCGTGTAG ATAACTACGA TACGGGAGGG CTTACCATCT 2760
   GGCCCCAGTG CTGCAATGAT ACCGCGAGAC CCACGCTCAC CGGCTCCAGA TTTATCAGCA 2820
   ATAAACCAGC CAGCCGGAAG GGCCGAGCGC AGAAGTGGTC CTGCAACTTT ATCCGCCTCC 2880
   ATCCAGTCTA TTAATTGTTG CCGGGAAGCT AGAGTAAGTA GTTCGCCAGT TAATAGTTTG 2940
   CGCAACGTTG TTGCCATTGC TGCAGGCATC GTGGTGTCAC GCTCGTCGTT TGGTATGGCT 3000
   TCATTCAGCT CCGGTTCCCA ACGATCAAGG CGAGTTACAT GATCCCCCAT GTTGTGCAAA 3060
   AAAGCGGTTA GCTCCTTCGG TCCTCCGATC GTTGTCAGAA GTAAGTTGGC CGCAGTGTTA 3120
   TCACTCATGG TTATGGCAGC ACTGCATAAT TCTCTTACTG TCATGCCATC CGTAAGATGC 3180
   TTTTCTGTGA CTGGTGAGTA CTCAACCAAG TCATTCTGAG AATAGTGTAT GCGGCGACCG 3240
   AGTTGCTCTT GCCCGGCGTC AACACGGGAT AATACCGCGC CACATAGCAG AACTTTAAAA 3300
   GTGCTCATCA TTGGAAAACG TTCTTCGGGG CGAAAACTCT CAAGGATCTT ACCGCTGTTG 3360
   AGATCCAGTT CGATGTAACC CACTCGTGCA CCCAACTGAT CTTCAGCATC TTTTACTTTC 3420
   ACCAGCGTTT CTGGGTGAGC AAAAACAGGA AGGCAAAATG CCGCAAAAAA GGGAATAAGG 3480
   GCGACACGGA AATGTTGAAT ACTCATACTC TTCCTTTTTC AATATTATTG AAGCATTTAT 3540
   CAGGGTTATT GTCTCATGAG CGGATACATA TTTGAATGTA TTTAGAAAAA TAAACAAATA 3600
   GGGGTTCCGC GCACATTTCC CCGAAAAGTG CCACCTGACG TCTAAGAAAC CATTATTATC 3660
   ATGACATTAA CCTATAAAAA TAGGCGTATC ACGAGGCCCT TTCGTCTTCA AG 3712
(2) INFORMATION FOR SEQ ID NO: 5:
   (i) SEQUENCE CHARACTERISTICS:
      (A) LENGTH: 3769 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 5:
   TTCAGGTAAA TTTGATGTAC ATCAAATGGT ACCCCTTGCT GAATCGTTAA GGTAGGCGGT 60
   AGGGCCCAGA TCTTAATCAT CCACAGGAGA CTTTCTGATG AAAAACCTTG ATTGTTGGGT 120
   CGACAACGAA GAAGACATCG ATGTTATCCT GAAAAAGTCT ACCATTCTGA ACTTGGACAT 180
   CAACAACGAT ATTATCTCCG ACATCTCTGG TTTCAACTCC TCTGTTATCA CATATCCAGA 240
   TGCTCAATTG GTGCCGGGCA TCAACGGCAA AGCTATCCAC CTGGTTAACA ACGAATCTTC 300
   TGAAGTTATC GTGCACAAGG CCATGGACAT CGAATACAAC GACATGTTCA ACAACTTCAC 360
   CGTTAGCTTC TGGCTGCGCG TTCCGAAAGT TTCTGCTTCC CACCTGGAAC AGTACGGCAC 420
   TAACGAGTAC TCCATCATCA GCTCTATGAA GAAACACTCC CTGTCCATCG GCTCTGGTTG 480
   GTCTGTTTCC CTGAAGGGTA ACAACCTGAT CTGGACTCTG AAAGACTCCG CGGGCGAAGT 540
   TCGTCAGATC ACTTTCCGCG ACCTGCCGGA CAAGTTCAAC GCGTACCTGG CTAACAAATG 600
   GGTTTTCATC ACTATCACTA ACGATCGTCT GTCTTCTGCT AACCTGTACA TCAACGGCGT 660
   TCTGATGGGC TCCGCTGAAA TCACTGGTCT GGGCGCTATC CGTGAGGACA ACAACATCAC 720
   TCTTAAGCTG GACCGTTGCA ACAACAACAA CCAGTACGTA TCCATCGACA AGTTCCGTAT 780
   CTTCTGCAAA GCACTGAACC CGAAAGAGAT CGAAAAACTG TATACCAGCT ACCTGTCTAT 840
   CACCTTCCTG CGTGACTTCT GGGGTAACCC GCTGCGTTAC GACACCGAAT ATTACCTGAT 900
   CCCGGTAGCT TCTAGCTCTA AAGACGTTCA GCTGAAAAAC ATCACTGACT ACATGTACCT 960
   GACCAACGCG CCGTCCTACA CTAACGGTAA ACTGAACATC TACTACCGAC GTCTGTACAA 1020
   CGGCCTGAAA TTCATCATCA AACGCTACAC TCCGAACAAC GAAATCGATT CTTTCGTTAA 1080
   ATCTGGTGAC TTCATCAAAC TGTACGTTTC TTACAACAAC AACGAACACA TCGTTGGTTA 1140
   CCCGAAAGAC GGTAACGCTT TCAACAACCT GGACAGAATT CTGCGTGTTG GTTACAACGC 1200
   TCCGGGTATC CCGCTGTACA AAAAAATGGA AGCTGTTAAA CTGCGTGACC TGAAAACCTA 1260
   CTCTGTTCAG CTGAAACTGT ACGACGACAA AAACGCTTCT CTGGGTCTGG TTGGTACCCA 1320
   CAACGGTCAG ATCGGTAACG ACCCGAACCG TGACATCCTG ATCGCTTCTA ACTGGTACTT 1380
   CAACCACCTG AAAGACAAAA TCCTGGGTTG CGACTGGTAC TTCGTTCCGA CCGATGAAGG 1440
   TTGGACCAAC GACGGGCCGG GGCCCTCTAG AGGATCCGAT ATCAAGCTTA CTAGTTAATG 1500
   ATCCGCTAGC CCGCCTAATG AGCGGGCTTT TTTTTCTCGG GCAGCGTTGG GTCCTGGCCA 1560
   CGGGTGCGCA TGATCGTGCT CCTGTCGTTG AGGACCCGGC TAGGCTGGCG GGGTTGCCTT 1620
   ACTGGTTAGC AGAATGAATC ACCGATACGC GAGCGAACGT GAAGCGACTG CTGCTGCAAA 1680
   ACGTCTGCGA CCTGAGCAAC AACATGAATG GTCTTCGGTT TCCGTGTTTC GTAAAGTCTG 1740
   GAAACGCGGA AGTCAGCGCT CTTCCGCTTC CTCGCTCACT GACTCGCTGC GCTCGGTCGT 1800
   TCGGCTGCGG CGAGCGGTAT CAGCTCACTC AAAGGCGGTA ATACGGTTAT CCACAGAATC 1860
   AGGGGATAAC GCAGGAAAGA ACATGTGAGC AAAAGGCCAG CAAAAGGCCA GGAACCGTAA 1920
   AAAGGCCGCG TTGCTGGCGT TTTTCCATAG GCTCCGCCCC CCTGACGAGC ATCACAAAAA 1980
   TCGACGCTCA AGTCAGAGGT GGCGAAACCC GACAGGACTA TAAAGATACC AGGCGTTTCC 2040
   CCCTGGAAGC TCCCTCGTGC GCTCTCCTGT TCCGACCCTG CCGCTTACCG GATACCTGTC 2100
   CGCCTTTCTC CCTTCGGGAA GCGTGGCGCT TTCTCAATGC TCACGCTGTA GGTATCTCAG 2160
   TTCGGTGTAG GTCGTTCGCT CCAAGCTGGG CTGTGTGCAC GAACCCCCCG TTCAGCCCGA 2220
   CCGCTGCGCC TTATCCGGTA ACTATCGTCT TGAGTCCAAC CCGGTAAGAC ACGACTTATC 2280
   GCCACTGGCA GCAGCCACTG GTAACAGGAT TAGCAGAGCG AGGTATGTAG GCGGTGCTAC 2340
   AGAGTTCTTG AAGTGGTGGC CTAACTACGG CTACACTAGA AGGACAGTAT TTGGTATCTG 2400
   CGCTCTGCTG AAGCCAGTTA CCTTCGGAAA AAGAGTTGGT AGCTCTTGAT CCGGCAAACA 2460
   AACCACCGCT GGTAGCGGTG GTTTTTTTGT TTGCAAGCAG CAGATTACGC GCAGAAAAAA 2520
   AGGATCTCAA GAAGATCCTT TGATCTTTTC TACGGGGTCT GACGCTCAGT GGAACGAAAA 2580
   CTCACGTTAA GGGATTTTGG TCATGAGATT ATCAAAAAGG ATCTTCACCT AGATCCTTTT 2640
   AAATTAAAAA TGAAGTTTTA AATCAATCTA AAGTATATAT GAGTAAACTT GGTCTGACAG 2700
   TTACCAATGC TTAATCAGTG AGGCACCTAT CTCAGCGATC TGTCTATTTC GTTCATCCAT 2760
   AGTTGCCTGA CTCCCCGTCG TGTAGATAAC TACGATACGG GAGGGCTTAC CATCTGGCCC 2820
   CAGTGCTGCA ATGATACCGC GAGACCCACG CTCACCGGCT CCAGATTTAT CAGCAATAAA 2880
   CCAGCCAGCC GGAAGGGCCG AGCGCAGAAG TGGTCCTGCA ACTTTATCCG CCTCCATCCA 2940
   GTCTATTAAT TGTTGCCGGG AAGCTAGAGT AAGTAGTTCG CCAGTTAATA GTTTGCGCAA 3000
   CGTTGTTGCC ATTGCTGCAG GCATCGTGGT GTCACGCTCG TCGTTTGGTA TGGCTTCATT 3060
   CAGCTCCGGT TCCCAACGAT CAAGGCGAGT TACATGATCC CCCATGTTGT GCAAAAAAGC 3120
   GGTTAGCTCC TTCGGTCCTC CGATCGTTGT CAGAAGTAAG TTGGCCGCAG TGTTATCACT 3180
   CATGGTTATG GCAGCACTGC ATAATTCTCT TACTGTCATG CCATCCGTAA GATGCTTTTC 3240
   TGTGACTGGT GAGTACTCAA CCAAGTCATT CTGAGAATAG TGTATGCGGC GACCGAGTTG 3300
   CTCTTGCCCG GCGTCAACAC GGGATAATAC CGCGCCACAT AGCAGAACTT TAAAAGTGCT 3360
   CATCATTGGA AAACGTTCTT CGGGGCGAAA ACTCTCAAGG ATCTTACCGC TGTTGAGATC 3420
   CAGTTCGATG TAACCCACTC GTGCACCCAA CTGATCTTCA GCATCTTTTA CTTTCACCAG 3480
   CGTTTCTGGG TGAGCAAAAA CAGGAAGGCA AAATGCCGCA AAAAAGGGAA TAAGGGCGAC 3540
   ACGGAAATGT TGAATACTCA TACTCTTCCT TTTTCAATAT TATTGAAGCA TTTATCAGGG 3600
   TTATTGTCTC ATGAGCGGAT ACATATTTGA ATGTATTTAG AAAAATAAAC AAATAGGGGT 3660
   TCCGCGCACA TTTCCCCGAA AAGTGCCACC TGACGTCTAA GAAACCATTA TTATCATGAC 3720
   ATTAACCTAT AAAAATAGGC GTATCACGAG GCCCTTTCGT CTTCAAGAA 3769
(2) INFORMATION FOR SEQ ID NO: 6:
   (i) SEQUENCE CHARACTERISTICS:
      (A) LENGTH: 1766 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iii) ANTI-SENSE: NO
   (v) FRAGMENT TYPE: internal
   (ix) FEATURE:
      (A) NAME/KEY: hinge region
      (B) LOCATION: 923..934
   (xi) SEQUENCE DESCRIPTION: SEQ ID NO: 6:
   GGGCGAAGTT CGTCAGATCA CTTTCCGCGA CCTGCCGGAC AAGTTCAACG CGTACCTGGC 60
   TAACAAATGG GTTTTCATCA CTATCACTAA CGATCGTCTG TCTTCTGCTA ACCTGTACAT 120
   CAACGGCGTT CTGATGGGCT CCGCTGAAAT CACTGGTCTG GGCGCTATCC GTGAGGACAA 180
   CAACATCACT CTTAAGCTGG ACCGTTGCAA CAACAACAAC CAGTACGTAT CCATCGACAA 240
   GTTCCGTATC TTCTGCAAAG CACTGAACCC GAAAGAGATC GAAAAACTGT ATACCAGCTA 300
   CCTGTCTATC ACCTTCCTGC GTGACTTCTG GGGTAACCCG CTGCGTTACG ACACCGAATA 360
   TTACCTGATC CCGGTAGCTT CTAGCTCTAA AGACGTTCAG CTGAAAAACA TCACTGACTA 420
   CATGTACCTG ACCAACGCGC CGTCCTACAC TAACGGTAAA CTGAACATCT ACTACCGACG 480
   TCTGTACAAC GGCCTGAAAT TCATCATCAA ACGCTACACT CCGAACAACG AAATCGATTC    540
   TTTCGTTAAA TCTGGTGACT TCATCAAACT GTACGTTTCT TACAACAACA ACGAACACAT    600
   CGTTGGTTAC CCGAAAGACG GTAACGCTTT CAACAACCTG GACAGAATTC TGCGTGTTGG    660
   TTACAACGCT CCGGGTATCC CGCTGTACAA AAAAATGGAA GCTGTTAAAC TGCGTGACCT    720
   GAAAACCTAC TCTGTTCAGC TGAAACTGTA CGACGACAAA AACGCTTCTC TGGGTCTGGT    780
   TGGTACCCAC AACGGTCAGA TCGGTAACGA CCCGAACCGT GACATCCTGA TCGCTTCTAA    840
   CTGGTACTTC AACCACCTGA AAGACAAAAT CCTGGGTTGC GACTGGTACT TCGTTCCGAC    900
   CGATGAAGGT TGGACCAACG ACGGGCCGGG GCCCTCTAGA GGATCCGATA TCAAGCTTAC    960
   TAGTTAATGA TCCGCTAGCC CGCCTAATGA GCGGGCTTTT TTTTCTCGGG CAGCGTTGGG   1020
   TCCTGGCCAC GGGTGCGCAT GATCGTGCTC CTGTCGTTGA GGACCCGGCT AGGCTGGCGG   1080
   GGTTGCCTTA CTGGTTAGCA GAATGAATCA CCGATACGCG AGCGAACGTG AAGCGACTGC   1140
   TGCTGCAAAA CGTCTGCGAC CTGAGCAACA ACATGAATGG TCTTCGGTTT CCGTGTTTCG   1200
   TAAAGTCTGG AAACGCGGAA GTCAGCGCTC TTCCGCTTCC TCGCTCACTG ACTCGCTGCG   1260
   CTCGGTCGTT CGGCTGCGGC GAGCGGTATC AGCTCACTCA AAGGCGGTAA TACGGTTATC   1320
   CACAGAATCA GGGGATAACG CAGGAAAGAA CATGTGAGCA AAAGGCCAGC AAAAGGCCAG   1380
   GAACCGTAAA AAGGCCGCGT TGCTGGCGTT TTTCCATAGG CTCCGCCCCC CTGACGAGCA   1440
   TCACAAAAAT CGACGCTCAA GTCAGAGGTG GCGAAACCCG ACAGGACTAT AAAGATACCA   1500
   GGCGTTTCCC CCTGGAAGCT CCCTCGTGCG CTCTCCTGTT CCGACCCTGC CGCTTACCGG   1560
   ATACCTGTCC GCCTTTCTCC CTTCGGGAAG CGTGGCGCTT TCTCAATGCT CACGCTGTAG   1620
   GTATCTCAGT TCGGTGTAGG TCGTTCGCTC CAAGCTGGGC TGTGTGCACG AACCCCCCGT   1680
   TCAGCCCGAC CGCTGCGCCT TATCCGGTAA CTATCGTCTT GAGTCCAACC CGGTAAGACA   1740
   CGACTTATCG CCACTGGCAG CAGCCA                                         1766
(2) INFORMATION FOR SEQ ID NO: 7:
   (i) SEQUENCE CHARACTERISTICS:
      (A) LENGTH: 1736 base pairs
      (B) TYPE: nucleic acid
      (D) TOPOLOGY: linear
   (ii) MOLECULE TYPE: DNA (genomic)
   (v) FRAGMENT TYPE: internal
   (xi) SEQUENCE DESCRIPTION: SEQ ID NO: 7:
   GGCGGCCTAC GCGATTGACG GCACGGCGGC GGGCGCCATG TACGGCAAGC ACATCACGCT     60
   GGTGTCCAGC GATTCAGGCC TGGGCGTGCG CCAGCTCGGC AGCCTGTCCT CGCCATCGGC    120
   CATCACCGTG TCGTCGCAGG GCGAAATCGC GCTGGGCGAC GCCACGGTCC AGCGCGGCCC    180
   GCTCAGCCTC AAGGGCGCGG GGGTCGTGTC GGCCGGCAAA CTGGCCTCCG GGGGGGGGGC    240
   GGTGAACGTC GCGGGCGGCG GGGCGGTGAA GATCGCGTCG GCCAGCAGCG TTGGAAACCT    300
   CGCGGTGCAA GGCGGCGGCA AGGTACAGGC CACGCTGTTG AATGCCGGGG GGACGTTGCT    360
   GGTGTCGGGC CGCCAGGCCG TCCAGCTTGG CGCGGCGAGC AGCCGTCAGG CGCTGTCCGT    420
   GAACGCGGGC GGCGCCCTCA AGGCGGACAA GCTGTCGGCG ACGCGACGGG TCGACGTGGA    480
   TGGCAAGCAG GCCGTCGCGC TGGGGTCGGC CAGCAGCAAT GCGCTGTCGG TGCGTGCCGG    540
   CGGCGCCCTC AAGGCGGGCA AGCTGTCGGC GACGGGGCGA CTGGACGTGG ACGGCAAGCA    600
   GGCCGTCACG CTGGGTTCGG TTGCGAGCGA CGGTGCGCTG TCGGTAAGCG CTGGCGGAAA    660
   CCTGCGGGCG AACGAATTGG TCTCCAGTGC CCAACTTGTG GTGCGTGGGC AGCGGGAGGT    720
   CGCGCTGGAT GACGCTTCGA GCGCACGCGG CATGACCGTG GTTGCCGCAG GAGCGCTGGC    780
   GGCCCGCAAC CTGCAGTCCA AGGGCGCCAT CGGCGTACAG GGTGGAGAGG CGGTCAGCGT    840
   GGCCAACGCG AACAGCGACG CGGAATTGCG CGTGCGCGGG CGCGGCCAGG TGGATCTGCA    900
   CGACCTGAGC GCAGCGCGCG GCGCGGATAT CTCCGGCGAG GGGCGCGTCA ATATCGGCCG    960
   TGCGCGCAGC GATAGCGATG TGAAGGTCTC CGGGCACGGC GCCTTGTCGA TCGATAGCAT   1020
   GACGGCCCTC GGTGCGATCG GCGTCCAGGC AGGCGGCAGC GTGTCGGCCA AGGATATGCG   1080
   CAGCCGTGGC GCCGTCACCG TCAGCGGCGG CGGCGCCGTC AACCTGGGCG ATGTCCAGTC   1140
   GGATGGGCAG GTCCGCGCCA CCAGCGCGGG CGCCATGACG GTGCGAGACG TCGCGGCTGC   1200
   CGCCGACCTT GCGCTGCAGG CGGGCGACGC GTTGCAGGCC GGGTTCCTGA AATCGGCCGG   1260
   TGCCATGACC GTGAACGGCC GCGATGCCGT GCGACTGGAT GGCGCGCACG CGGGCGGGCA   1320
   ATTGCGGGTT TCCAGCGACG GGCAGGCTGC GTTGGGCAGT CTCGCGGCCA AGGGCGAGCT   1380
   GACGGTATCG GCCGCGCGCG CGGCGACCGT GGCCGAGTTG AAGTCGCTGG ACAACATCTC   1440
   CGTGACGGGC GGCGAACGCG TGTCGGTTCA GAGCGTCAAC AGCGCGTCCA GGGTCGCCAT   1500
   TTCGGCGCAC GGCGCGCTGG ATGTAGGCAA GGTTTCCGCC AAGAGCGGTA TCGGGCTCGA   1560
   AGGCTGGGGC GCGGTCGGAG CGGACTCCCT CGGTTCCGAC GGCGCGATCA GCGTGTCCGG   1620
   GCGCGATGCG GTCAGGGTCG ATCAAGCCCG CAGTCTTGCC GACATTTCGC TGGGGGCGGA   1680
   AGGCGGCGCC ACGCTGGGCG CGGTGGAGGC CGCCGGTTCG ATCGACGTGC GCGGCG       1736